(19) Europäisches Patentamt
European Patent Office
Office européen des brevets

(11) EP 0 536 087 B1

(12) **FASCICULE DE BREVET EUROPEEN**

(45) Date de publication et mention de la délivrance du brevet: **24.04.1996 Bulletin 1996/17**

(21) Numéro de dépôt: **92810736.6**

(22) Date de dépôt: **30.09.1992**

(51) Int. Cl.$^6$: **B65D 83/00**, D06M 15/647, D06M 13/342, D06M 15/227, D04H 1/00, D04H 3/00, A61M 31/00, C02F 1/00, A01G 29/00, A61K 9/00, A01N 25/34

(54) **Système-réservoir pour diffusion prolongée d'un principe actif**

Behälter zur langsamen Abgabe eines Wirkstoffes

System-container for the prolonged diffusion of an active component

(84) Etats contractants désignés:
**AT BE CH DE DK ES FR GB IT LI LU NL SE**

(30) Priorité: **03.10.1991 FR 9112186**

(43) Date de publication de la demande:
**07.04.1993 Bulletin 1993/14**

(73) Titulaire: **HOLVIS HOLZSTOFF SA**
**CH-4051 Bâle (CH)**

(72) Inventeurs:
- **Ehret, Philippe**
  **F-68320 Fortschwihr (FR)**
- **Rougeot, Christophe**
  **F-68000 Colmar (FR)**
- **Brochart, Hervé**
  **F-68310 Wittelsheim (FR)**
- **Stamm, André**
  **F-67210 Griesheim (FR)**

(74) Mandataire: **Frossard, Michel, Dr. et al**
**A. Braun, Braun, Héritier, Eschmann AG,**
**Patentanwälte**
**Holbeinstrasse 36-38**
**CH-4051 Basel (CH)**

(56) Documents cités:
**EP-A- 0 281 236**
**EP-A- 0 340 993**
**DE-A- 3 813 773**
**FR-A- 2 638 059**
**JP-A- 5 779 809**
**US-A- 4 207 893**
**US-A- 4 434 153**

- **DATABASE WPIL Week 8943, Derwent Publications Ltd., London, GB; AN 89-312491**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 536 087 B1

Printed by Rank Xerox (UK) Business Services
2.11.2/3.4

## Description

La présente invention a pour objet un système-réservoir pour la libération et la diffusion prolongées de principes actifs dans un milieu aqueux ou soumis à l'action de l'eau, ainsi qu'un procédé de fabrication dudit. Le nouveau système-réservoir permet une libération lente, constante, étalée dans le temps de façon linéaire et s'étendant, en général, sur une durée d'une semaine au moins.

Il est fait, au moins pour la partie destinée à se trouver au contact de l'eau, d'un nontissé constitué de monofilaments continus ou/et de microfibres en polymères synthétiques thermoplastiques et façonné, ou combiné avec une feuille d'un matériau imperméable, en forme de poche fermée contenant un ou des principes actifs solubles dans l'eau ou qui peuvent être rendus solubles dans le milieu aqueux auquel ils sont destinés.

Pour un principe actif donné, une cinétique de libération linéaire telle que voulue est obtenue en ajustant en conséquence le degré d'hydrophilie du nontissé et les dimensions du nontissé définissant la surface d'échange entre le volume intérieur du système-réservoir et le milieu aqueux auquel il est destiné.

La libération et la diffusion prolongées de principes actifs dans certains milieux ont déjà fait l'objet de multiples essais et des solutions de nature très différente ont été proposées pour y parvenir.

Ainsi, le brevet US 4 207 893 décrit un dispositif tubulaire de mise en liberté d'un fluide contenant un principe actif. Le fluide est enfermé dans un tube déformable dont les extrémités présentent deux petites ouvertures d'écoulement. Ce tube est entouré d'un laminé faut d'un matériau absorbant et d'un polymère hydrophile et gonflable. L'ensemble est inséré dans un tube rigide formé d'un matériau perméable ou microporeux. Placé dans un milieu biologique tel qu'une cavité corporelle, le dispositif absorbe de l'eau, le polymère hydrophile se gonfle et exerce donc une pression sur le tube déformable qui contient le fluide.

Un dispositif de construction analogue, mais d'un mode d'action différent est décrit dans le document GB 2 048 710. Le tube déformable comporte à l'une de ses extrémités un canal capillaire pour l'écoulement du fluide; le laminé absorbant et gonflant est remplacé par une composition d'activité osmotique élevée. Lorsque le dispositif est placé dans un milieu aqueux tel qu'une cavité corporelle, la composition attire l'eau par osmose, à travers le tube rigide perméable ou microporeux, elle augmente ainsi de volume et fait pression sur le tube déformable contenant le fluide.

Ces dispositifs sont d'une construction compliquée, ce qui se répercute sur les procédés et les coûts de fabrication. Ces désavantages ont fait rechercher des systèmes plus simples et plus économiques. Les solutions adoptées communément sont fondées sur l'incorporation du principe actif dans une matière polymère servant de matrice, et sa diffusion à partir de cette matrice; elles diffèrent les unes des autres par la composition chimique de la matrice et par le mode d'action du milieu ambiant sur celle-ci.

Selon le document EP 281 236, la matrice est un élastomère de silicone réticulé, au sein duquel une phase liquide polaire ou hydrophile contenant le principe actif est dispersée en microcapsules d'un diamètre inférieur à 20 micromètres et forme une émulsion d'eau dans l'huile, grâce à un agent de dispersion. Lorsque la matrice a une résistance mécanique élevée, elle peut être utilisée comme implant dans un organisme vivant, dans le cas contraire, pour application dermique du principe actif; le degré de réticulation influence en sens inverse la vitesse de libération du principe actif.

Une matrice d'un autre genre est décrite dans le brevet US 4 434 153. Elle consiste en un polymère hydrophile capable d'augmenter de volume par absorption d'eau et de se désintégrer par hydrolyse ou érosion biologique. La désintégration de cette matrice dans l'estomac d'un animal ou d'un être humain libère une multitude de pilules d'un diamètre d'environ 0,5 à 1 cm, qui contiennent le principe actif. Celui-ci, sous forme d'un noyau, est enfermé dans une enveloppe consistant en un mélange d'une cire et d'un triglycéride ou en une autre matière capable de régler la vitesse de mise en liberté; le principe actif est libéré par diffusion à travers l'enveloppe ou par désintégration de celle-ci, érosion biologique ou rupture par la pression osmotique. L'effet retard résulte donc de deux enrobages superposés, qui se désintègrent l'un après l'autre.

Selon le brevet US 4 209 607, on obtient des polyesteramides par réaction de bis-oxamidodiols avec des acides dicarboxyliques ou leurs diesters, par exemple des diester: oxaliques, succiniques, subériques ou téréphtaliques. Les dérivés d'esters oxaliques sont sensibles à l'hydrolyse; ils peuvent être utilisés pour fabriquer des pièces absorbables pour la chirurgie, notamment des fils pour suture. Les dérivés d'esters succiniques et autres sont résistants à l'eau; ils peuvent être utilisés pour des sutures chirurgicales non absorbables et en tant que fibres textiles.

La publication de brevet JP-A-1 229 857 décrit une membrane de nontissé consistant en filaments d'alcool polyvinylique et en fibres de polyoléfines, de polyamides ou de polyesters, qui est capable de fixer, par affinité chimique, des microorganismes, des bactéries, des spores etc. Cette membrane peut être utilisée en microbiologie, notamment pour le traitement des eaux usées et des eaux résiduelles de la fabrication de produits alimentaires ou d'enzymes.

La demande de brevet EP 340 993 décrit une poche dont une paroi est formée d'un nontissé caractérisé par une dimension des pores et une porosité Gurley de valeurs données. La poche contient un liquide, par exemple une solution de détergent, qui diffuse à travers la paroi seulement lorsque la surface externe de celle-ci est mouillée par le même liquide.

Selon la publication de brevet JP-A-57/079 809, on peut prolonger la durée d'action d'un engrais d'effet lent en le mélangeant à un matériau de nature minérale ou organique capable de retenir l'eau et en plaçant la matrice ainsi formée dans une poche faite d'un nontissé d'une épaisseur de 0,1 à 0,3 mm et d'une perméabilité au gaz d'une valeur donnée.

Il est clair que les systèmes de matrice ci-dessus ne sauraient suggérer un système-réservoir en forme de poche fermée, consistant en un nontissé perméable à l'eau parce qu'ajusté à un certain degré d'hydrophilie, ainsi que l'objet de l'invention a été défini ci-dessus.

Un élément essentiel de l'invention est le caractère prolongé et constant de la diffusion du principe actif que doit assurer le nontissé et qu'il doit maintenir durant la période d'utilisation. Si l'on considère le fait que le système-réservoir est destiné à être utilisé dans un milieu aqueux ou dans un milieu dans lequel l'eau peut servir de véhicule à un principe actif, il est clair que les propriétés de diffusion ne doivent pas être modifiées lorsque le système est soumis à l'action de l'eau, ce qui entraînerait l'altération du caractère linéaire de la cinétique de libération.

Or, on a trouvé qu'il est possible d'ajuster durablement le degré d'hydrophilie d'un nontissé à un certain niveau, choisi d'après la solubilité dans l'eau du principe actif visé, par imprégnation au moyen de certains groupes particuliers de composes.

L'invention a donc pour objet un système-réservoir façonné en forme de poche fermée et contenant un principe actif pour diffusion prolongée et constante, dans un milieu aqueux ou soumis à l'action de l'eau, du principe actif qui est soluble ou qui peut être rendu soluble dans ledit milieu, caractérisé en ce qu'il est fait d'un nontissé constitué de monofilaments continus ou/et de microfibres en polymères synthétiques thermoplastiques, ledit nontissé étant traité soit, s'il est de nature hydrophobe, par un polysiloxane ou un polymère à base de polysiloxane ou par un sel d'ammonium quaternaire de type amphotère, soit, s'il est de nature hydrophile, par un composé perfluoré ou par un agent d'hydrofugation à base d'une résine acrylique et de paraffine, et en ce que, pour un principe actif donné, le degré d'hydrophilie conféré ou laissé au nontissé et les dimensions de la poche définissant la suface d'échange entre le volume intérieur et le milieu aqueux auquel il est destiné sont variables et ajustés pour une cinétique de libération linéaire telle que voulue.

L'invention a également pour objet un procédé de fabrication du système-réservoir mentionné ci-dessus, caractérisé en ce que, à partir d'une ou de deux bandes d'un nontissé constitué de microfibres ou monofilaments continus en polymères synthétiques thermoplastiques et traité soit, s'il est de nature hydrophobe, par un polysiloxane ou un polymère à base de polysiloxane ou par un sel d'ammonium quaternaire de type amphotère, soit, s'il est de nature hydrophile, par un composé perfluoré ou par un agent d'hydrofugation à base d'une résine acrylique et de paraffine, on prépare une poche de forme rectangulaire ou carrée, la largeur de la bande ou des deux bandes déterminant la largeur de la poche, soit en pliant en deux une bande, soit en superposant l'une à l'autre deux bandes, dans l'un et l'autre cas dans le sens longitudinal de manière à ce que les bords se recouvrent, soit l'un l'autre dans le cas du pliage, soit deux par deux dans le cas de la superposition, dans ce dernier cas on effectue sur l'un des côtés une soudure des bords l'un sur l'autre, on effectue ensuite dans l'un et l'autre cas des soudures dans le sens transversal, à distances égales, la distance entre deux soudures transversales déterminant la longueur de la poche, on introduit le au les principes actifs et,le cas échéant le ou les excipients ou/et additifs, dans les poches par le côté resté ouvert entre deux soudures transversales, au moyen d'un dispositif de dosage, on effectue une soudure des bords l'un à l'autre sur le côté resté ouvert, afin de fermer les poches, et on sépare les poches les unes des autres par coupure sur les soudures transversales.

Lorsqu'il s'agit d'un nontissé de nature hydrophobe, il s'est avéré que l'imprégnation, soit par des polysiloxanes ou des polymères à base de polysiloxane, soit par des sels d'ammonium quaternaire de type amphotère est propre à conférer à ce nontissé un caractère hydrophile permanent. Ces constatations sont hautement surprenantes. En effet, les polysiloxanes sont connus comme des composés de nature hydrophobe (Römpp Chemie Lexikon, 9ème éd. vol. 5,page 4169, Georg Thieme Verlag, Stuttgart New York 1992) et non pas comme des agents d'hydrophilie, et c'est pourtant bien ce qui ressort des essais présentés aux exemples suivants. Le fait que des fibres ou filaments hydrophobes puissent devenir mouillables, c'est-à-dire perméables à l'eau, par l'effet d'un polysiloxane ne peut être expliqué à ce jour. D'autre part, vu la nature hydrophile (ibidem, page 3731) des sels d'ammonium quaternaire, on devait s'attendre à ce qu'ils soient redissous et éliminés (lessivés) du nontissé lorsque le système-réservoir est soumis à l'action de l'eau. Pourtant, il n'en est rien, ainsi qu'il ressort des essais de laboratoire: ces sels restent fixés durablement sur le nontissé, si l'imprégnation a été effectuée correctement.

Lorsqu'il s'agit d'un nontissé de nature hydrophile, c'est-à-die mouillable par l'eau, l'eau du milieu aqueux peut diffuser librement à travers le nontissé. La libération du principe actif s'effectuera donc sans autre, à une vitesse dépendant essentiellement de sa solubilité dans l'eau. Aussi est-il impératif, si l'on veut obtenir une libération du principe actif qui soit lente, constante, étalée dans le temps de façon linéaire, de diminuer durablement le degré d'hydrophilie du nontissé et, dans ce but, de l'imprégner par un agent hydrofugeant. Mais un tel agent, s'il se fixe de façon durable sur les fibres ou les filaments, devrait les rendre entièrement hydrophobes. Or, on a trouvé, contre toute attente, que les composés perfluorés et les agents d'hydrofugation à base d'une résine acrylique et de paraffine se fixent sur les fibres de façon durable, mais sans toutefois masquer entièrement le caractère hydrophile foncier du nontissé.

L'invention est décrite ci-après plus en détail. Sur les figures jointes 1 à 5 et 10, l'axe des abscisses représente le temps, en heures ou en jours, l'axe des ordonnées représente la quantité de principe actif mise en liberté, en mg.

Le nontissé utilisé pour fabriquer les poches est obtenu selon les procédés connus, en général par application du procédé de soufflage de microfibres, dit melt-blown, ou du procédé de filage de monofilaments continus, dit spun. On obtient ainsi soit un nontissé filé et soufflé, soit un nontissé filé.

Le nontissé est constitué, de préférence, de microfibres ou monofilaments continus en polyoléfines, polyesters ou polyamides, notamment en polypropylène, polyéthylène, polybutylènetéréphtalate ou/et polyéthylènetéréphtalate.

A titre d'exemple, le polypropylène, le polyéthylène et le polybutylènetéréphtalate sont fortement hydrophobes: des voiles de nontissé réalisés avec ces polymères sont imperméables à l'eau si un agent d'hydrophilie n'a pas été utilisé au préalable pour les rendre mouillables. A l'inverse, la plupart des polyamides sont très hydrophiles; c'est le cas entre autres pour le polyhexaméthylène-adipamide. Ils requièrent donc, pour être utilisés dans le système-réservoir selon l'invention, un traitement préalable par un agent capable d'abaisser de façon permanente le degré d'hydrophilie. Quant aux polyesters, le degré d'hydrophilie peut, suivant leur composition, les rapprocher de l'un ou de l'autre groupe.

La figure 1 montre, dans le cas d'un voile de nontissé en polypropylène, l'influence de la nature chimique du composé utilisé pour conférer au nontissé le degré d'hydrophilie requis pour la libération du principe actif. L'imprégnation a été réalisée par une solution alcoolique à 0,01 % de polyéther polydiméthylsiloxane (PPS) et de N-alcoylaminobétaïne (NAAB), respectivement. Comme principe actif, on a utilisé 1000 g de paracétamol. Le témoin, un voile semblable mais non imprégné, est représenté par les signes carrés alignés sur l'axe des abscisses; il montre qu'il n'y a aucune libération du principe actif.

Comme polysiloxanes, on peut utiliser, entre autres des polysiloxanes organomodifiés hydrosolubles et des copolymères de polysiloxane et de polyéther tels que ceux qui sont décrits, par exemple dans Ullmanns Encyklopädie der technischen Chemie, 4e édition, volume 21, page 534 et suiv. (Verlag Chemie GmbH, Weinheim/RFA 1982).

Par sels d'ammonium quaternaire de type amphotère on entend des composés d'ammonium quaternaire dont l'un des substituants alcoyle porte un groupe acide (ou anionique), notamment un groupe carboxylique, un groupe sulfonique, un groupe sulfurique, un groupe phosphonique ou un groupe phosphorique. Ces substances sont nommées aussi bétaïnes, par exemple les N-alcoylaminobétaïnes. On peut donc utiliser entre autres des chlorures ou des méthylsulfates de dialcoyldiméthylammonium ou de 1,2-dialcoyl-3-méthylimidazolinium dont un substituant alcoyle porte l'un des groupes acides mentionnés ci-dessus. On se reportera à ce sujet à Ullmann's Encyclopedia of Industrial Chemistry, 5e édition, volume A8, page 350 et suiv. (VCH Verlagsgesellschaft mbH, Weinheim/RFA 1987).

Comme exemples particuliers des deux groupes de produits mentionnés ci-dessus, on citera d'un part le polyéther-polydiméthylsiloxane copolymère HANSA FINISH 1601, agent tensioactif non ionique fabriqué par H.T.C., Hansa Textilchemie GmbH, Oyten bei Bremen/RFA, et distribué sous la marque ABIL B 8851 par Goldschmidt France SA, F - 78180 Montigny le Bretonneux. Le nom chimique de ce produit est le diméthiconepolyol, répondant à la formule suivante:

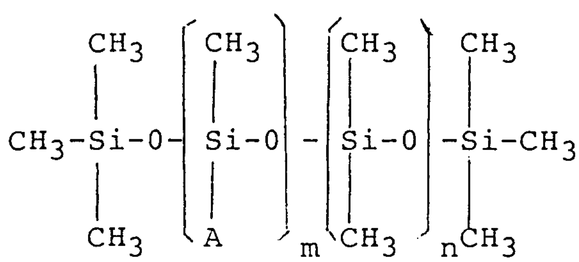

avec m = environ 5, n = environ 60 et A = $(CH_2)_3\text{-}O\text{-}(CH_2CH_2O)_x\text{-}(CH_2CH_2CH_2O)_yH$ (x et y non définis).

On citera, d'autre part, le produit KK 71006/1 fabriqué par Sandoz Produkte AG, CH-4002 Bâle, dont la formule développée est la suivante:

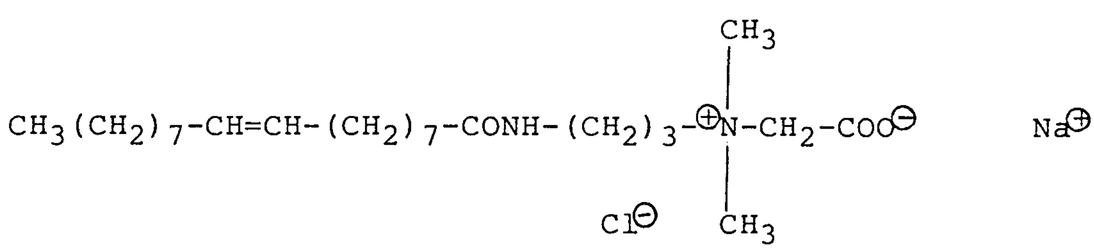

Comme exemple particulier d'un agent d'hydrofugation, on citera les produits PARAFFION $R_1$ et PARAFFION $R_2$ fabriqués par Röhm GmbH Chemische Fabrik, D-6100 Darmstadt 1 (RFA), l'un et l'autre à base d'une résine acrylique et de paraffine, mais qui diffèrent par le degré de leur viscosité.

L'imprégnation du nontissé par l'un des produits mentionnés ci-dessus peut s'effectuer avantageusement en traitant le nontissé au moyen d'une solution dudit produit dans un solvant approprié tel que l'eau, un alcool, diol ou polyol aliphatique inférieur, par exemple le méthanol, l'éthanol, l'isopropanol, l'éthylèneglycol, le propylèneglycol, le diéthylèneglycol, un éther ou un mélange d'un de ceux-ci avec l'eau. Un mode de faire approprié consiste à appliquer la solution par foulardage ou léchage, puis séchage du nontissé.

Dans des essais comparatifs visant à définir l'influence, sur la cinétique de libération, de la concentration de la solution utilisée pour l'imprégnation, on a mis en oeuvre des solutions alcooliques à 0,0025 % et 0,01 %, respectivement, d'un polyéther-polydiméthylsiloxane (PPS) et d'une N-alcoylaminobétaïne (NAAB); la poche de nontissé était telle que décrite à l'exemple 1. Or, les figures 2 et 3 ne montrent aucune différence significative des quantités de principe actif (sulfadimidine, 300 mg) libérées au bout de 20 heures. Cette absence d'influence de la concentration confirme que c'est bien la nature chimique et non pas la quantité de l'agent de surface utilisé qui est déterminante pour une cinétique de libération d'ordre zéro.

Après l'imprégnation, il est recommandé de laver à l'eau le nontissé pour en éliminer une fraction de l'agent d'imprégnation en excès par rapport à la quantité que le nontissé est capable de fixer durablement et qui retarderait l'établissement des conditions d'équilibre dans le milieu aqueux.

Quant au principe actif, il est soluble ou peut être rendu soluble à un degré suffisant dans l'environnement auquel il est destiné, c'est-à-dire en général dans un milieu aqueux tel que la panse d'un ruminant, les installations de traitement des eaux, notamment des eaux destinées aux nécessités humaines. On entend par là les installations de conditionnement de l'eau pour la consommation, les installations sanitaires, les installations d'épuration des eaux usées, celles qui servent à la préparation de l'eau des piscines et bains publics, des bassins des piscicultures etc. Outre les milieux proprement aqueux mentionnés ci-dessus, on peut envisager aussi des milieux qui sont soumis de temps en temps à l'action de l'eau, en particulier les sols et les plants utilisés en horticulture et en arboriculture.

Le caractère linéaire de la cinétique de libération a pour effet notoire d'exclure les fluctuations de concentration du milieu aqueux en principe actif, fluctuations que l'on observe d'habitude lorsque le traitement est réalisé par des administrations réitérées.

Selon le milieu et le but que l'on se propose d'atteindre, le principe actif peut être, notamment, un antibiotique, un antifongique, un antiparasitaire tel qu'un anticoccidien ou un anthelmintique, un antiseptique, une vitamine, un oligoélément, un facteur de croissance, un produit phytosanitaire, un engrais, un algicide ou une association de deux ou plusieurs d'entre eux.

Les domaines d'application sont, entre autres:

- la médecine vétérinaire en ce qui concerne l'administration d'antibiotiques, d'antiparasitaires, de vitamines, etc... aussi bien à titre préventif que curatif. Dans ce cas, les antiinfectieux et antiparasitaires sont destinés en particulier à éviter les risques d'infection lors du transport et de la mise en pâturage;
- le traitement des eaux tel que la lutte contre les algues, les champignons et bactéries;
- l'horticulture et l'arboriculture, où l'on peut exposer une plante ou un arbre aux effets d'un engrais ou d'un facteur de croissance sans risquer un dommage aux racines ou au tronc.

Afin de modifier la disponibilité du ou des principes actifs par rapport au milieu aqueux environnant ou soumis à l'action de l'eau, le système-réservoir peut renfermer, en plus, un ou des excipients et/ou additifs. Ceux-ci doivent, soit favoriser la mise à disposition du milieu environnant de principes actifs dont le coefficient de solubilité dans l'eau est nul ou jugé insuffisant, soit freiner la mise à disposition dudit milieu de principes actifs dont le coefficient de solubilité est jugé trop élevé. On utilisera à cette fin soit des agents tensioactifs (voir Ullman's Encyclopedia of Industrial Chemistry, 5e édition, volume A8, page 338 et suiv. (VCH Verlagsgesellschaft mbH, Weinheim/RFA 1987), soit des agents gonflants formant des gels matriciels retardant la diffusion du ou des principes actifs dans le milieu environnant (polyacrylates, amidon modifié par greffage etc). Un exemple desdits polyacrylates est le produit FAVOR SAB fabriqué par Stockhausen France, F-60100 Creil; il s'agit là d'un sel de copolymère réticulé et greffé d'acide polyacrylique et d'un polyol.

D'autres excipients et/ou additifs permettent, par exemple, d'accélérer le processus de diffusion de l'eau du milieu environnant vers l'intérieur du système-réservoir; ce sont par exemple des sels pourvus d'un pouvoir osmotique, notamment le chlorure de sodium.

Le système-réservoir peut également renfermer une grille constituée d'un polymère semi-synthétique biodégradable et qui lui confère un encombrement stérique tel que, placé dans la panse d'un ruminant, il ne puisse être régurgité.

D'autre part, la vitesse de libération augmente lorsque la surface d'échange entre le milieu auquel le système-réservoir est soumis et l'intérieur du sachet augmente, ainsi qu'il ressort de la figure 4. Les quantités de sulfadimidine libérées au bout de 20 heures par des systèmes-réservoirs chargés à 300 mg et présentant des surfaces de diffusion

de 50 et 100 $cm^2$ ont été, respectivement, de 131 ± 9 mg et 252 ± 18 mg. On voit que, lorsque la surface de diffusion double, la quantité de principe actif libérée augmente dans des proportions voisines (1,93 fois).

La charge de principe actif n'a, tous autres paramètres restant identiques, pas d'influence sur la cinétique de libération, aussi longtemps que des conditions de saturation en principe actif se maintiennent à l'intérieur du système-réservoir. La figure 5, pour des charges en sulfadimidine de 300 mg et 1000 mg, respectivement, montre sensiblement la même courbe.

La durée d'action du système dépend donc de la surface d'échange de la poche et de la quantité de principe actif incorporé dans la poche.

Les mécanismes régissant la diffusion du principe actif dans le modèle proposé sont en étroite relation avec la loi de Fick. Ceci permet d'expliquer notamment le caractère linéaire de la cinétique de libération et d'en fixer les limites en appliquant la modification suivante de la loi de FICK:

$$\frac{dq}{dt} = - D \times S \times \frac{dc}{dx}$$

$\frac{dq}{dt}$ = vitesse de diffusion

D = coefficient de diffusion (fonction du coefficient de solubilité du principe actif et de l'intensité du traitement hydrophile)

S = surface d'échange

$\frac{dc}{dx}$ = gradient de concentration entre les milieux intérieur et environnant

Le caractère linéaire de la vitesse de libération du principe actif n'est effectif que si la différence de concentration en principe actif entre le milieu aqueux auquel le système-réservoir est soumis et le milieu constitué par l'intérieur du système est constante.

En effet, la fraction non dissoute du principe actif remplace au fur et à mesure de la diffusion, la part de principe actif dissoute qui est libérée. La concentration en principe actif à l'intérieur de la poche se maintiendra donc à saturation jusqu'à épuisement de la fraction non dissoute du principe actif, c'est-à-dire pendant toute la durée d'action du système.

Il convient donc de définir la charge de principe actif à introduire dans la poche lors de la fabrication, en fonction de la vitesse de diffusion du principe actif, de la durée d'action désirée ainsi que de la surface d'échange du système-réservoir.

Un procédé appliqué pour fabriquer et remplir de principe actif le système-réservoir est caractérisé en ce que, à partir d'une ou de deux bandes d'un nontissé constitué de microfibres ou monofilaments continus en polymères synthétiques thermoplastiques et ajusté à un degré d'hydrophilie défini comme décrit ci-dessus, on prépare une poche de forme rectangulaire ou carrée, la largeur de la bande ou des deux bandes déterminant la largeur de la poche, soit en pliant en deux une bande, soit en superposant l'une à l'autre deux bandes, dans l'un et l'autre cas dans le sens longitudinal de manière à ce que les bords se recouvrent, soit l'un l'autre dans le cas du pliage, soit deux par deux dans le cas de la superposition, dans ce dernier cas on effectue sur l'un des côtés une soudure des bords l'un sur l'autre, on effectue ensuite dans l'un et l'autre cas des soudures dans le sens transversal, à distances égales, la distance entre deux soudures transversales déterminant la longueur de la poche, on introduit le ou les principes actifs ainsi que, le cas échéant, le ou les excipients ou/et additifs dans les poches par le côté resté ouvert entre deux soudures transversales, au moyen d'un dispositif de dosage, on effectue une soudure des bords l'un sur l'autre sur le côté resté ouvert, afin de fermer les poches, et on les sépare les unes des autres par coupure sur les soudures transversales.

Les soudures sont effectuées avantageusement par l'action de chaleur ou d'ultrasons ou par collage. Le dispositif de dosage peut être, en particulier, une pompe doseuse ou toute autre machine de remplissage et de conditionnement de sachets utilisée dans l'industrie alimentaire. La fabrication sera effectuée, par exemple, dans un atelier observant les prescriptions connues sous le nom de "Good Manufacturing Practice".

Les systèmes-réservoirs obtenus sont emmagasinés, de préférence, à l'abri de l'humidité, avantageusement dans des boites en carton ou en polyéthylène.

**Essais de dissolution**

Les essais de dissolution in vitro ont été réalisés à la Faculté de Pharmacie de l'Université de Strasbourg, au Laboratoire de Pharmacotechnie, sur une chaîne de dissolution automatisée comprenant:

- un appareil de dissolution équipé d'un système de prélèvement automatique
- un spectrophotomètre pour le domaine UV-visible

- un ordinateur avec ses accessoires, pourvu de logiciels permettant une intégration et une interprétation directes, notamment statistique, des résultats.

Mode opératoire:

Conforme à la deuxième édition de la Pharmacopée Européenne, vol. 5, chapitre 4 (juillet 1987), essai intitulé "Essai de dissolution des formes orales solides" selon la méthode dite de la palette tournante.

- Milieu de dissolution: eau distillée
- Volume: 1000 ml
- Vitesse de rotation des palettes: 25 tours/min.
- Sachets fixés sur les palettes de l'appareil à dissolution
- Dosage pour spectrophotométrie: la longueur d'onde sélectionnée est fonction du principe actif mis en jeu
- Durée de l'essai: variable

Les figures 6 à 10 illustrent le caractère prolongé de la diffusion de principes actifs in vitro à partir d'un système-réservoir conforme à la présente invention. L'échelle des abscisses représente le temps (t) exprimé en heures sur les figures 6, 8 et 9, en jours sur les figures 7 et 10; l'échelle des ordonnées représente sur les figures 6 à 9 le pourcentage de la quantité initiale de principe actif libéré au temps t (%, masse/masse), sur la figure 10 la quantité libérée par jour, en mg.

Les figures 6 à 9 représentent la cinétique de libération de la sulfadimidine - figures 6 et 7 - et du paracétamol - figures 8 et 9 - à partir de systèmes-réservoirs dont les caractéristiques sont décrites, respectivement, aux exemples 1 à 4.

Exemple 1

Des poches de format 10 x 10 cm sont réalisées à partir d'un nontissé 100% polypropylène obtenu par le procédé dit melt-blown. Les caractéristiques du polypropylène sont les suivantes:

- grammage: 20 g/$m^2$
- résistance, sens machine: 6,6 N/inch
  sens transversal: 2,9 N/inch
- allongement sens machine: 21,2 %
  sens transversal: 53,3 %
- absorption d'eau: 605 %

Cette capacité de fixation d'eau est obtenue en imprégnant le polypropylène au moyen d'une solution éthanolique de HANSA FINISH 1601, un polysiloxane organomodifié de la Société H.T.C., Hansa Textilchemie GmbH; aucune mention de traitement hydrophile n'est faite par le fabricant. Une fois le nontissé imprégné par léchage (vitesse du rouleau gommeur: 8,8 m/min., vitesse de défilement du nontissé sur le rouleau gommeur: 15 m/min.), il est séché et façonné comme décrit précédemment.

A l'intérieur de chaque poche, 0,5 g de sulfadimidine pesés exactement sont introduits. L'ensemble est alors soumis à l'essai de dissolution mentionné ci-dessus.

La concentration de la solution éthanolique en HANSA FINISH 1601 est, exprimée en pourcent (masse/volume):

pour le lot A = 0,5%
pour le lot B = 1,0%
pour le lot C = 1,5%
pour le lot D = 1,0%

La figure 6 montre que:

- le lot D libère 100% de 0,5 g = 0,5 g de sulfadimidine après 60 heures
- les lots B et C libèrent 100% de 0,5 g de sulfadimidine après 98 heures
- le lot A libère 90% de 0,5 g = 0,45 g de sulfadimidine après 160 heures.

Pour les 4 lots, la cinétique de diffusion de la sulfadimidine est à caractère prolongé et linéaire.

Exemple 2

Des systèmes-réservoir de format 7 x 7 cm sont réalisés au moyen de l'assemblage à chaud, par passage sur une calandre, de deux voiles nontissés:

a) un voile nontissé obtenu par le procédé dit melt-blown 100% polypropylène:

- grammage: 20g/$m^2$
- rendu hydrophile par une solution éthanolique à 0,01% de HANSA FINISH 1601
  Le mode de faire est identique à celui de l'exemple 1.

b) un voile nontissé obtenu par le procédé dit spun 100% polypropylène:

- grammage: 70 g/$m^2$
- rendu hydrophile par une solution éthanolique de 1% de HANSA FINISH 1601
  Le protocole est identique à celui de l'exemple 1.

Le voile nontissé décrit en a) assure les propriétés de diffusion prolongée du principe actif, le voile nontissé décrit en b) assure les propriétés de résistance mécanique du système-réservoir.

On introduit dans chaque système-réservoir du lot A (voir exemple 1) 5,0 g de sulfadimidine.

La figure 7 montre que le lot A a libéré 13% de 5,0 g = 650 mg de sulfadimidine après 20 jours, selon une cinétique de diffusion à caractère prolongé et linéaire.

Exemple 3

Les caractéristiques des systèmes-réservoir réalisés sont rigoureusement les mêmes que celles des systèmes-réservoir décrits dans l'exemple 2. Par contre, le principe actif étudié est différent: 1,0 g de paracétamol est introduit dans chaque système-réservoir du lot A.

La figure 8 montre que le lot A libère 24% de 1,0 g = 240 mg de paracétamol en 7 heures selon une cinétique de diffusion à caractère prolongé et linéaire.

Exemple 4

1) Lot A

Des systèmes-réservoir de 7 x 7 cm sont réalisés au moyen de l'assemblage à chaud, par passage sur une calandre, de deux voiles nontissés:

a) un voile nontissé obtenu par le procédé dit melt-blown 100% polyéthylène:

- grammage: 20 g/$m^2$
- traité hydrophile par une solution éthanolique à 0,01% de HANSA FINISH 1601
  Le mode de faire est identique à celui de l'exemple 1.

b) un voile nontissé identique à celui décrit dans l'exemple 2, b).

2) Lot B

Des systèmes-réservoir de même format sont réalisés au moyen de l'assemblage à chaud, par passage sur une calandre, de deux voiles nontissés:

a) un voile nontissé obtenu par le procédé dit melt-blown 100% polybutylènetéréphtalate:

- grammage: 20 g/$m^2$
- non traité hydrophile.

b) un voile nontissé identique à celui décrit dans l'exemple 2, b).

1,0 g de paracétamol est introduit dans chacun des systèmes-réservoir des lots A et B.

La figure 9 montre que le lot A libère 83% de 1,0 g = 830 mg de paracétamol en 20 heures et que le lot B libère 92% de 1,0 g = 920 mg de paracétamol en 20 heures selon une cinétique de diffusion à caractère prolongé et linéaire pour les deux lots.

Exemple 5

On prépare un sachet carré de 10 x 10 cm à partir d'un voile de 70 $g/m^2$ constitué de filaments de polypropylène de 30 µm de diamètre, destiné à former la paroi externe du sachet, et d'un voile de 20 $g/m^2$ constitué de filaments de polypropylène d'un diamètre de 3 µm, destiné à former la paroi interne. Ce dernier est traité par léchage au moyen d'une solution alcoolique de N-alcoylaminobétaïne (NAAB) à 0,01 %. Après ce traitement, les deux voiles sont assemblés à chaud par passage sur une calandre, mais en veillant à ne pas effectuer la soudure des bords respectifs sur l'un des côtés du sachet. Par le côté resté ouvert, on introduit dans le sachet une grille en polyéthylène de 5 mm d'ouverture de maille (mesh), puis le principe actif, à savoir 1000 mg de paracétanol. Après soudure du côté resté ouvert, on soumet le sachet à l'essai de dissolution décrit ci-dessus. La cinétique de libération est illustrée par la figure 1.

Exemple 6

La figure 10 représente la quantité de sulfaméthazine libérée chaque jour, pendant un laps de temps de 22 jours, à partir d'une poche de nontissé en polypropylène, d'une surface de 100 $cm^2$ (renforcée par un voile nontissé de même surface, tel que décrit à l'exemple 2b), renfermant 10 g de sulfaméthazine et traitée au préalable par un copolymère de polyéther et polydiméthylsiloxane. La mise en liberté du principe actif s'effectue selon une cinétique d'ordre zéro, à raison de 245 ± 13 mg (n = 5) par jour pendant 22 jours ($R^2$ = 0,9995). La quantité libérée chaque jour est suffisante pour prévenir la coccidiose chez les agneaux.

**Revendications**

1. Système-réservoir façonné en forme de poche fermée et contenant un principe actif pour diffusion prolongée et constante, dans un milieu aqueux ou soumis à l'action de l'eau, du principe actif qui est soluble ou qui peut être rendu soluble dans ledit milieu, caractérisé en ce qu'il est fait d'un nontissé constitué de monofilaments continus ou/et de microfibres en polymères synthétiques thermoplastiques, ledit nontissé étant traité soit, s'il est de nature hydrophobe, par un polysiloxane ou un polymère à base de polysiloxane ou par un sel d'ammonium quaternaire de type amphotère, soit, s'il est de nature hydrophile, par un composé perfluoré ou par un agent d'hydrofugation à base d'une résine acrylique et de paraffine, et en ce que, pour un principe actif donné, le degré d'hydrophilie conféré ou laissé au nontissé et les dimensions de la poche définissant la surface d'échange entre le volume intérieur et le milieu aqueux auquel il est destiné sont variables et ajustés pour une cinétique de libération linéaire telle que voulue.

2. Système-réservoir selon la revendication 1, caractérisé en ce que le nontissé est constitué de microfibres ou monofilaments en polyoléfines, polyesters ou polyamides.

3. Système-réservoir selon la revendication 2, caractérisé en ce que le nontissé est constitué de microfibres ou monofilaments en polypropylène, polyéthylène, polybutylènetéréphtalate ou polyéthylènetéréphtalate.

4. Système-réservoir selon la revendication 3, caractérisé en ce que le nontissé est constitué de microfibres ou monofilaments en polypropylène.

5. Système-réservoir selon la revendication 1, caractérisé en ce que le principe actif est associé à un ou des excipients ou additifs permettant d'en régler la disponibilité par rapport au milieu aqueux auquel il est destiné.

6. Système-réservoir selon la revendication 1, caractérisé en ce que le principe actif est un antibiotique, un antifongique, un antiparasitaire, un antiseptique, une vitamine, un oligoélément, un facteur de croissance, un produit phytosanitaire, un engrais, un algicide ou une association de deux ou plusieurs d'entre eux.

7. Procédé de fabrication du système-réservoir selon la revendication 1, caractérisé en ce que, à partir d'une ou de deux bandes d'un nontissé constitué de microfibres ou monofilaments continus en polymères synthétiques thermoplastiques et traité soit, s'il est de nature hydrophobe, par un polysiloxane ou un polymère a base de polysiloxane ou par un sel d'ammonium quaternaire de type amphotère, soit, s'il est de nature hydrophile, par un composé perfluoré ou par un agent d'hydrofugation à base d'une résine acrylique et de paraffine, on prépare une poche de forme rectangulaire ou carrée, la largeur de la bande ou des deux bandes déterminant la largeur de la poche, soit en pliant en deux une bande, soit en superposant l'une à l'autre deux bandes, dans l'un et l'autre cas dans le sens

longitudinal de manière à ce que les bords se recouvrent, soit l'un l'autre dans le cas du pliage, soit deux par deux dans le cas de la superposition, dans ce dernier cas on effectue sur l'un des côtés une soudure des bords l'un sur l'autre, on effectue ensuite dans l'un et l'autre cas des soudures dans le sens transversal, à distances égales, la distance entre deux soudures transversales déterminant la longueur de la poche, on introduit le ou les principes actifs et, le cas échéant le ou les excipients ou/et additifs, dans les poches par le côté resté ouvert entre deux soudures transversales, au moyen d'un dispositif de dosage, on effectue une soudure des bords l'un a l'autre sur le côté resté ouvert, afin de fermer les poches, et on sépare les poches les unes des autres par coupure sur las soudures transversales.

**8.** Procédé selon la revendication 7, caractérisé en ce que le traitement est effectué par imprégnation du nontissé au moyen d'une solution dudit produit dans l'eau, un alcool, diol ou polyol aliphatique inférieur ou un éther de ceux-ci, puis séchage du nontissé

**9.** Procédé selon la revendication 7, caractérisé en ce que les soudures sont effectuées par l'action de chaleur ou d'ultrasons au par collage.

**10.** Utilisation du système-réservoir selon la revendication 1 dans un milieu aqueux, ou soumis a l'action ce l'eau, de la panse d'un ruminant, d'installations de traitement des eaux à usage industriel ou des eaux destinées aux nécessités humaines, et de sols ou de plants utilisés en horticulture ou arboriculture.

**Claims**

**1.** Reservoir system fashioned into the shape of a closed bag and containing an active principle, for prolonged and constant diffusion, in an environment which is aqueous or subjected to the action of water, of said active principle which is soluble or which can be made soluble in the said environment, characterized in that it is made of a nonwoven consisting of continuous monofilaments or/and of microfibers made of thermoplastic synthetic polymers, the said nonwoven being treated either, if it is hydrophobic in nature, with a polysiloxane or a polymer based on polysiloxane or with a quaternary ammonium salt of amphoteric type, or, if it is hydrophilic in nature, with a perfluorinated compound or with a water-repelling agent based on an acrylic resin and paraffin, and in that, for a given active principle, the degree of hydrophily conferred or left on the nonwoven and the dimensions of the nonwoven which define the surface area for exchange between the internal volume and the aqueous environment for which it is intended are variable and adjusted for the desired linear release kinetics.

**2.** Reservoir system according to Claim 1, characterized in that the nonwoven consists of microfibers or monofilaments made of polyolefins, polyesters or polyamides.

**3.** Reservoir system according to Claim 2, characterized in that the nonwoven consists of microfibers or monofilaments made of polypropylene, polyethylene, poly(butylene terephthalate) or poly(ethylene terephthalate).

**4.** Reservoir system according to Claim 3, characterized in that the nonwoven consists of microfibers or monofilaments made of polypropylene.

**5.** Reservoir system according to Claim 1, characterized in that the active principle is combined with one or more excipients or additives making it possible to adjust its availability with respect to the aqueous environment for which it is intended.

**6.** Reservoir system according to Claim 1, characterized in that the active principle is an antibiotic, an antifungal agent, an antiparasitic agent, an antiseptic, a vitamin, a trace element, a growth factor, a plant protection product, a fertilizer, an algicide or a combination of two or more of them.

**7.** Process for the manufacture of the reservoir system according to Claim 1, characterized in that, from one or two strips of a nonwoven consisting of microfibers or continuous monofilaments made of thermoplastic synthetic polymers and treated either, if it is hydrophobic in nature, with a polysiloxane or polymer based on polysiloxane or with a quaternary ammonium salt of amphoteric type, or, if it is hydrophilic in nature, with a perfluorinated compound or with a water-repelling agent based on an acrylic resin and paraffin, a bag of rectangular or square shape is prepared, the width of the strip or the two strips determining the width of the bag, either by folding a strip in two, or by superimposing two strips one on the other, in both cases in the longitudinal direction so that the edges lie over each other, either one over the other in the case of folding or in twos in the case of superimposing; in the latter case the edges are welded together on one of the sides, in both cases weldings are then carried out in the transverse direction, at

equal distances, the distance between two transverse weldings determining the length of the bag, the active principle(s) and, if appropriate, the excipient(s) or/and additive(s) are introduced into the bags through the side which remains open between two transverse welds, by means of a dosimeter, the edges on the side which remains open are welded together, in order to close the bags, and they are separated from each other by cutting along the transverse welds.

8. Process according to Claim 7, characterized in that the treatment is carried out by impregnation of the nonwoven with a solution of the said product in water, a lower aliphatic alcohol, diol or polyol, or an ether of these, and then drying of the nonwoven.

9. Process according to Claim 7, characterized in that the welds are produced by the action of heat or ultrasound or by bonding.

10. Use of the reservoir system according to Claim 1 in an environment which is aqueous or subjected to the action of water, consisting of the rumen or a ruminant, plants for the treatment of water for industrial use or water intended for human requirements, and soils or culture beds used in horticulture or arboriculture.

**Patentansprüche**

1. Reservoirsystem, das als geschlossene Tasche ausgebildet ist und einen Wirkstoff enthält, zur verlängerten und anhaltenden Diffusion des Wirkstoffes in einem wässrigen oder der Einwirkung von Wasser ausgesetzten Medium, wobei der Wirkstoff in besagtem Medium löslich ist oder löslich gemacht werden kann, dadurch gekennzeichnet, dass es aus einem Vlies besteht, das aus kontinuierlichen Monofilamenten und/oder aus Stapelfasern von thermoplastischen synthetischen Polymeren gebildet ist, wobei besagtes Vlies, wenn es hydrophob ist, mit einem Polysiloxan oder einem von Polysiloxan abgeleiteten Polymer oder mit einem quaternären Ammoniumsalz von amphoterem Typus bzw., wenn es hydrophil ist, mit einer Perfluorverbindung oder einem Hydrophobierungsmittel auf Acrylharz- und Paraffinbasis behandelt ist und wobei je nach Wirkstoff dem Vlies ein bestimmter Grad an Hydrophilie verliehen oder belassen wird und besagter Grad sowie die Abmessungen der die Austauschfläche zwischen dem Innenvolumen und dem wässrigen Zielmedium darstellenden Tasche veränderlich und auf die gewünschte lineare Freisetzungskinetik eingestellt sind.

2. Reservoirsystem nach Anspruch 1, dadurch gekennzeichnet, dass das Vlies aus Stapelfasern oder Monofilamenten aus Polyolefinen, Polyestern oder Polyamiden besteht.

3. Reservoirsystem nach Anspruch 2, dadurch gekennzeichnet, dass das Vlies aus Stapelfasern oder Monofilamenten aus Polypropylen, Polyethylen, Polybutylenterephthalat oder Polyethylenterephthalat besteht.

4. Reservoirsystem nach Anspruch 3, dadurch gekennzeichnet, dass das Vlies aus Stapelfasern oder Monofilamenten aus Polypropylen besteht.

5. Reservoirsystem nach Anspruch 1, dadurch gekennzeichnet, dass der Wirkstoff mit einem oder mehreren Hilfsstoffen oder Zusatzstoffen vermengt ist, welche dessen Verfügbarkeit gegenüber dem wässrigen Zielmedium einzustellen vermögen.

6. Reservoirsystem nach Anspruch 1, dadurch gekennzeichnet, dass der Wirkstoff ein Antibiotikum, ein Fungizid, ein Pestizid, ein Antiseptikum, ein Vitamin, ein Oligoelement, ein Wachstumsfaktor, ein phytosanitäres Produkt, ein Düngemittel, ein Algizid oder eine Kombination von zwei oder mehreren derselben ist.

7. Verfahren zur Herstellung des Reservoirsystems nach Anspruch 1, dadurch gekennzeichnet, dass, ausgehend von einem oder zwei Streifen Vlies, wobei letzteres aus Stapelfasern oder kontinuierlichen Monofilamenten von thermoplastischen synthetischen Polymeren besteht und, wenn es hydrophob ist, mit einem Polysiloxan oder einem von Polysiloxan abgeleiteten Polymer oder mit einem quaternären Ammoniumsalz von amphoterem Typus bzw., wenn es hydrophil ist, mit einer Perfluorverbindung oder einem Hydrophobierungsmittel auf Acrylharz- und Paraffinbasis behandelt worden ist, eine rechteckige oder quadratische Tasche, deren Breite durch die Breite des oder der zwei Streifen bestimmt wird, gebildet wird, indem ein Streifen hälftig über sich selbst gefaltet wird oder die zwei Streifen übereinander gelegt werden und zwar in beiden Fällen in Längsrichtung, so dass die Ränder beim Falten einander überdecken bzw. beim Uebereinanderlegen paarweise einander überdecken, im letzteren Fall diese Ränder auf der einen Seite miteinander verschweisst werden, danach in beiden Fällen Schweissnähte in Querrichtung in gleichen Abständen ausgeführt werden, wobei die Länge der Tasche durch den Abstand zwischen zwei

Schweissnähten bestimmt wird, der oder die Wirkstoffe und gegebenenfalls das oder die Hilfsstoffe und/oder Zusatzstoffe durch die zwischen zwei querliegenden Schweissnähten offen gebliebene Seite in die Taschen mittels einer Dosiervorrichtung eingebracht werden, an der offen gebliebenen Seite zum Verschliessen der Taschen die Ränder miteinander verschweisst werden und die Taschen mittels Durchschneiden auf den querliegenden Schweissnähten voneinander getrennt werden.

**8.** Verfahren nach Anspruch 7, dadurch gekennzeichnet, dass die Behandlung durch Imprägnieren des Vlieses mittels einer Lösung des besagten Produktes in Wasser, einem niederen aliphatischen Alkohol, Diol oder Polyol oder einem Ether derselben und danach Trocknen des Vlieses durchgeführt wird.

**9.** Verfahren nach Anspruch 7, dadurch gekennzeichnet, dass die Schweissnähte durch Einwirkung von Wärme oder Ultraschall oder durch Verkleben durchgeführt werden.

**10.** Verwendung des Reservoirsystems nach Anspruch 1 in einem wässrigen oder der Einwirkung von Wasser ausgesetzten Medium des Pansens eines Wiederkäuers, der Aufbereitungsanlagen für Wasser zur Verwendung in der Industrie oder für den täglichen Lebensbedarf und der in Gartenbau oder Landwirtschaft verwendeten Böden bzw. des Pflanzengutes.

Fig. 1

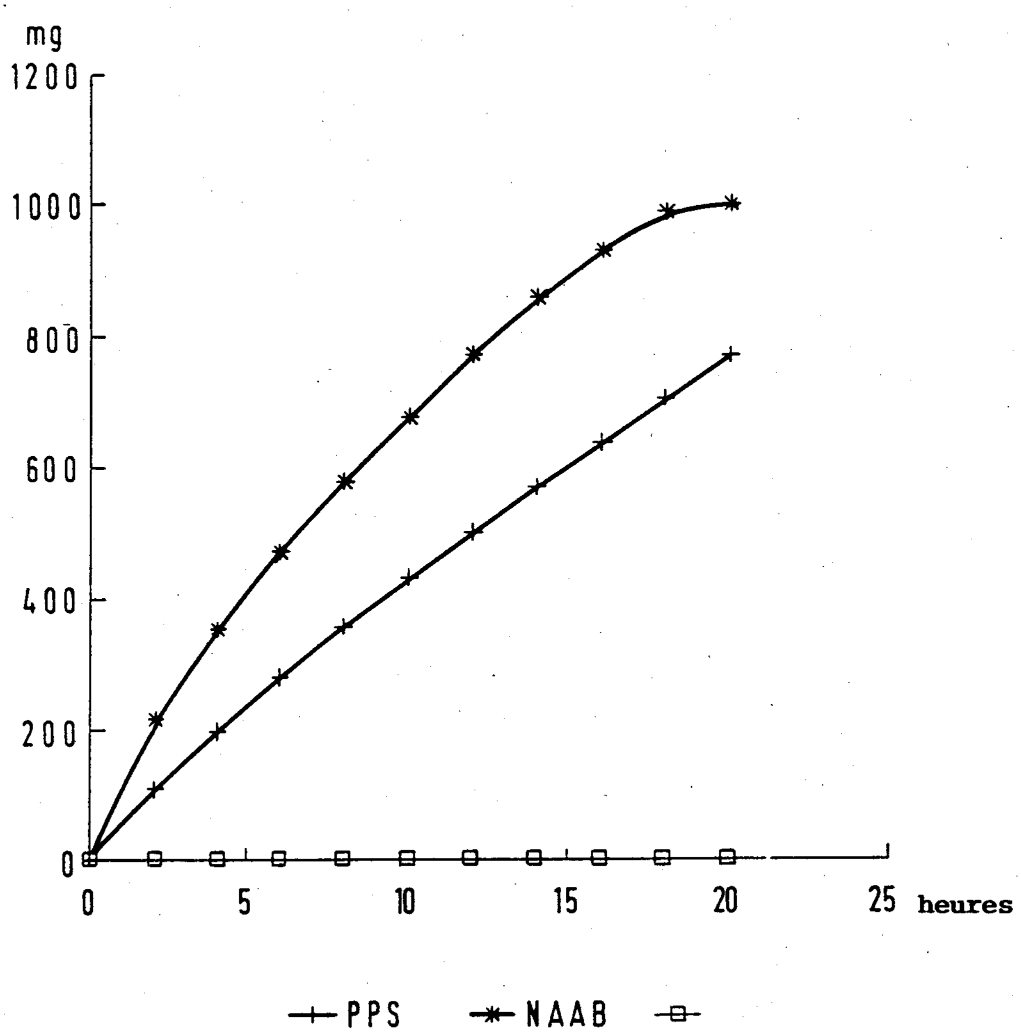

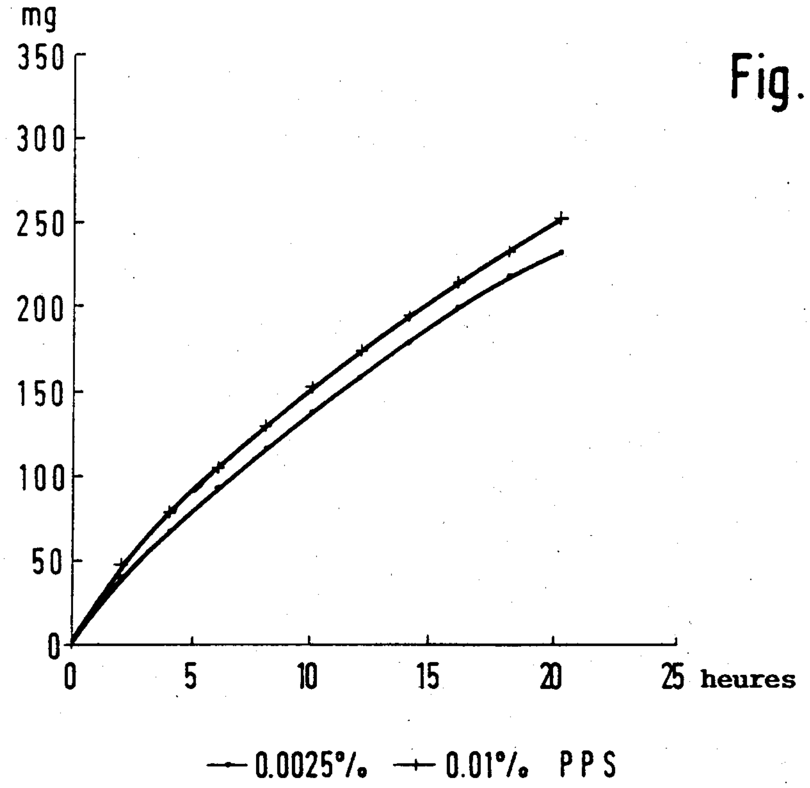
mg
350
300
250
200
150
100
50
0
0
5
10
15
20
25 heures
—•— 0.0025°/。 —+— 0.01°/。 PPS

Fig.2

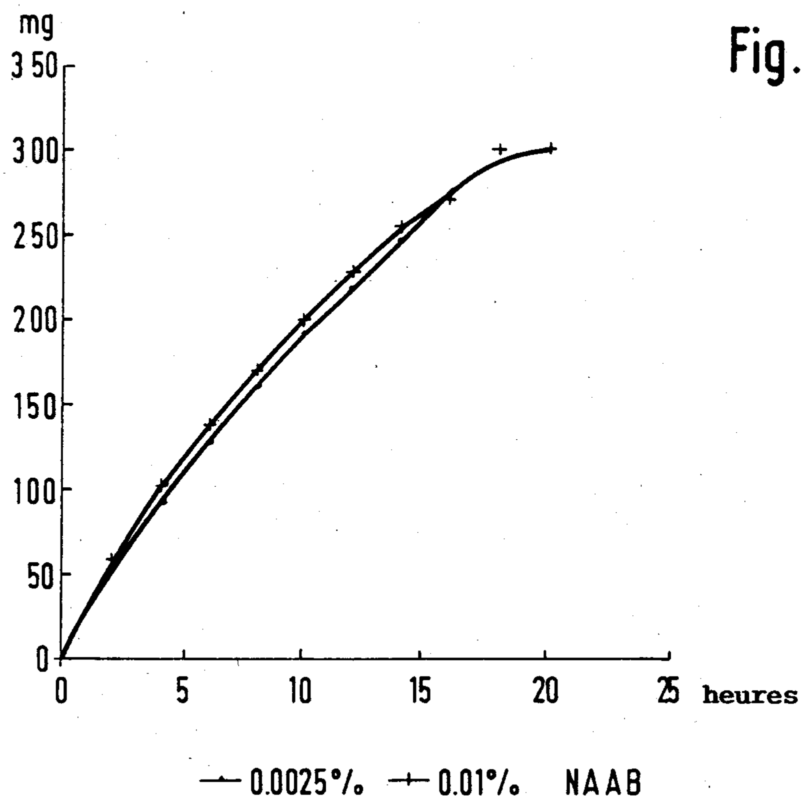
mg
350
300
250
200
150
100
50
0
0
5
10
15
20
25 heures
—•— 0.0025°/。 —+— 0.01°/。 NAAB

Fig.3

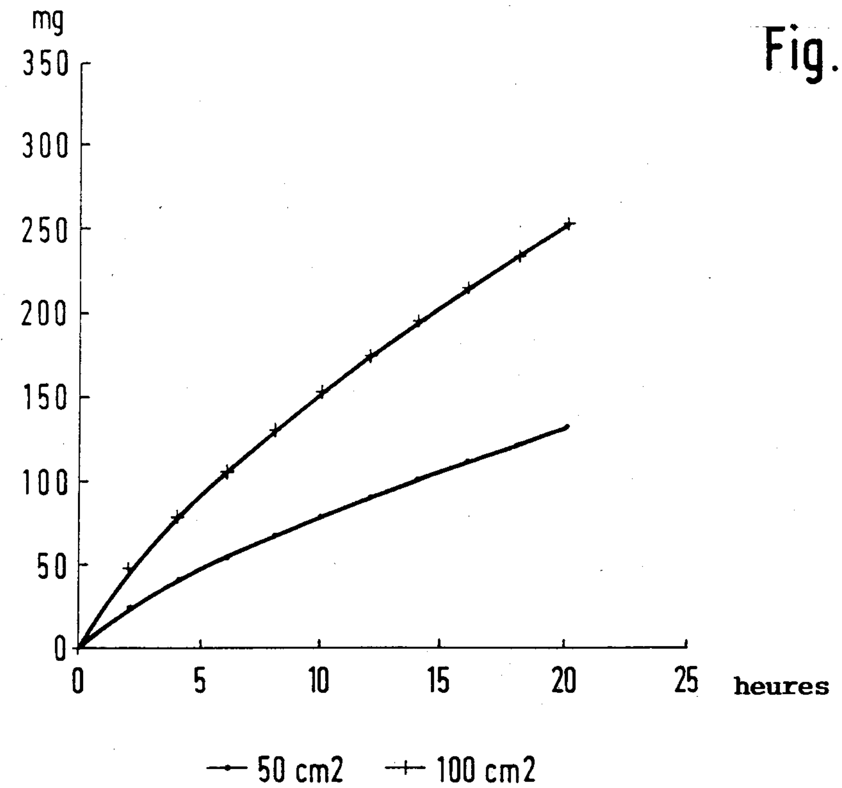
mg
350
300
250
200
150
100
50
0
0
5
10
15
20
25
heures
50 cm2
100 cm2

Fig.4

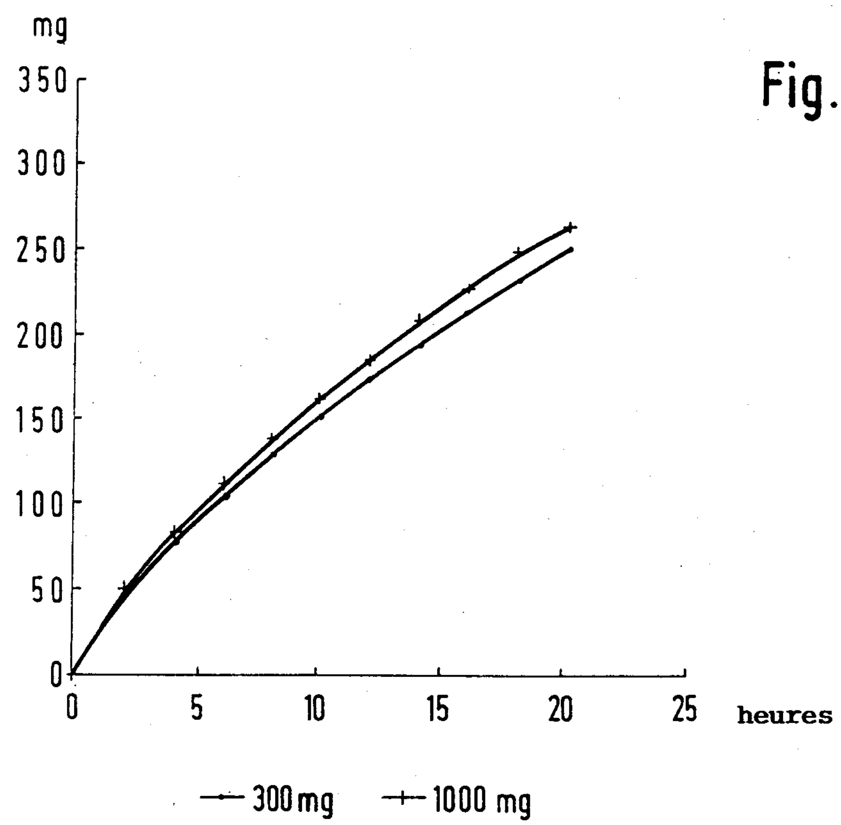
mg
350
300
250
200
150
100
50
0
0
5
10
15
20
25
heures
300mg
1000 mg

Fig.5

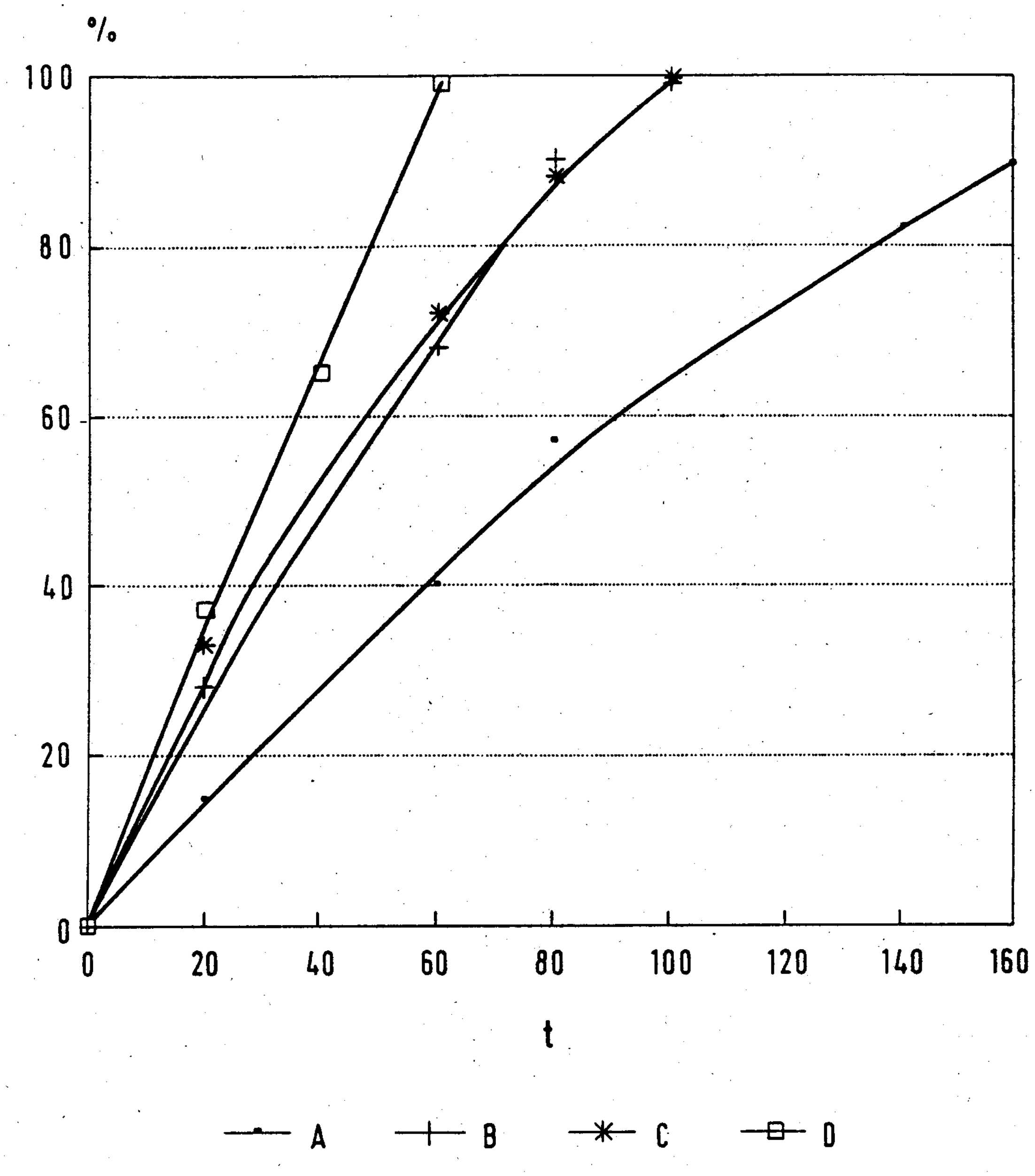


Fig.6

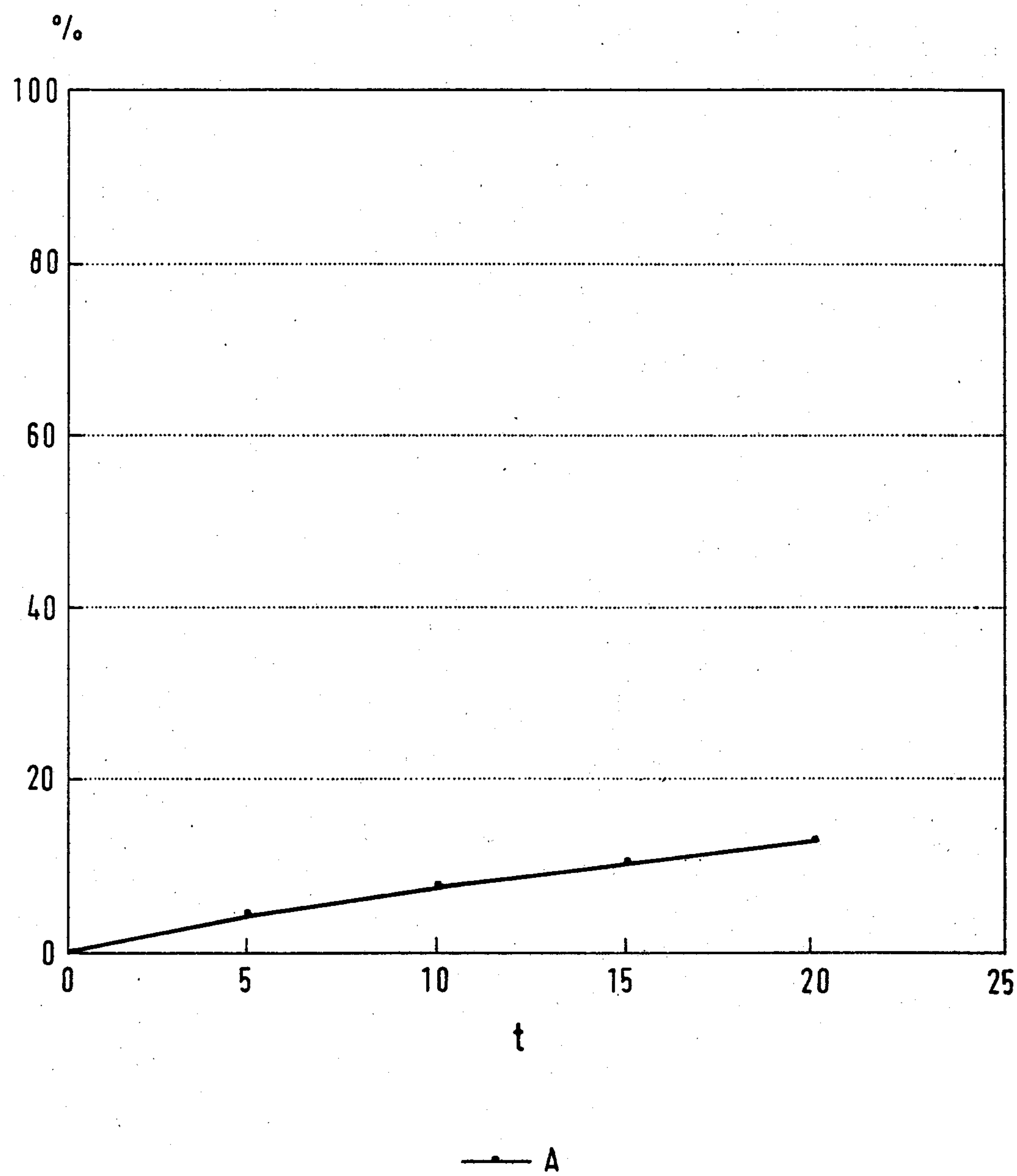


Fig. 7

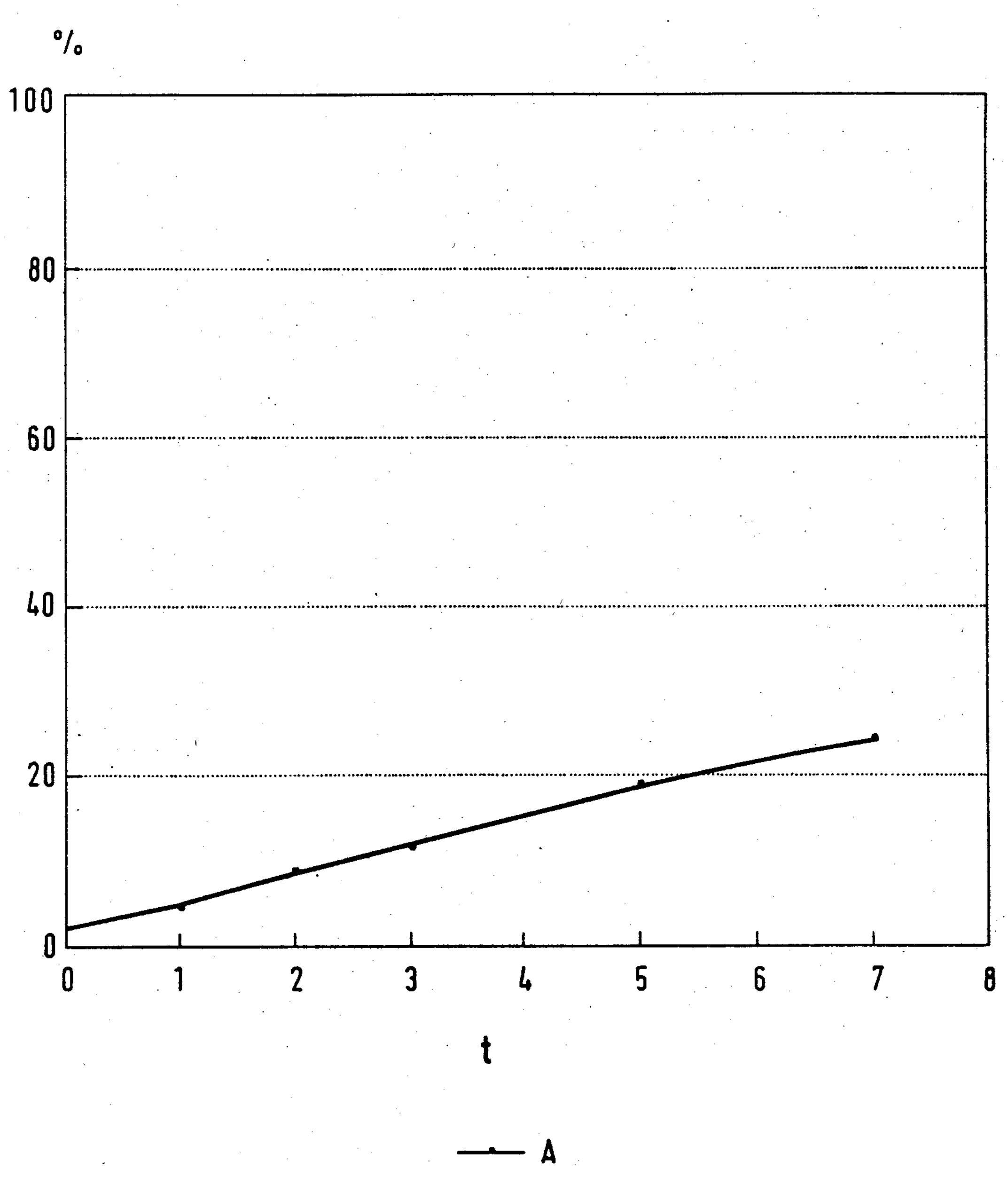


Fig. 8

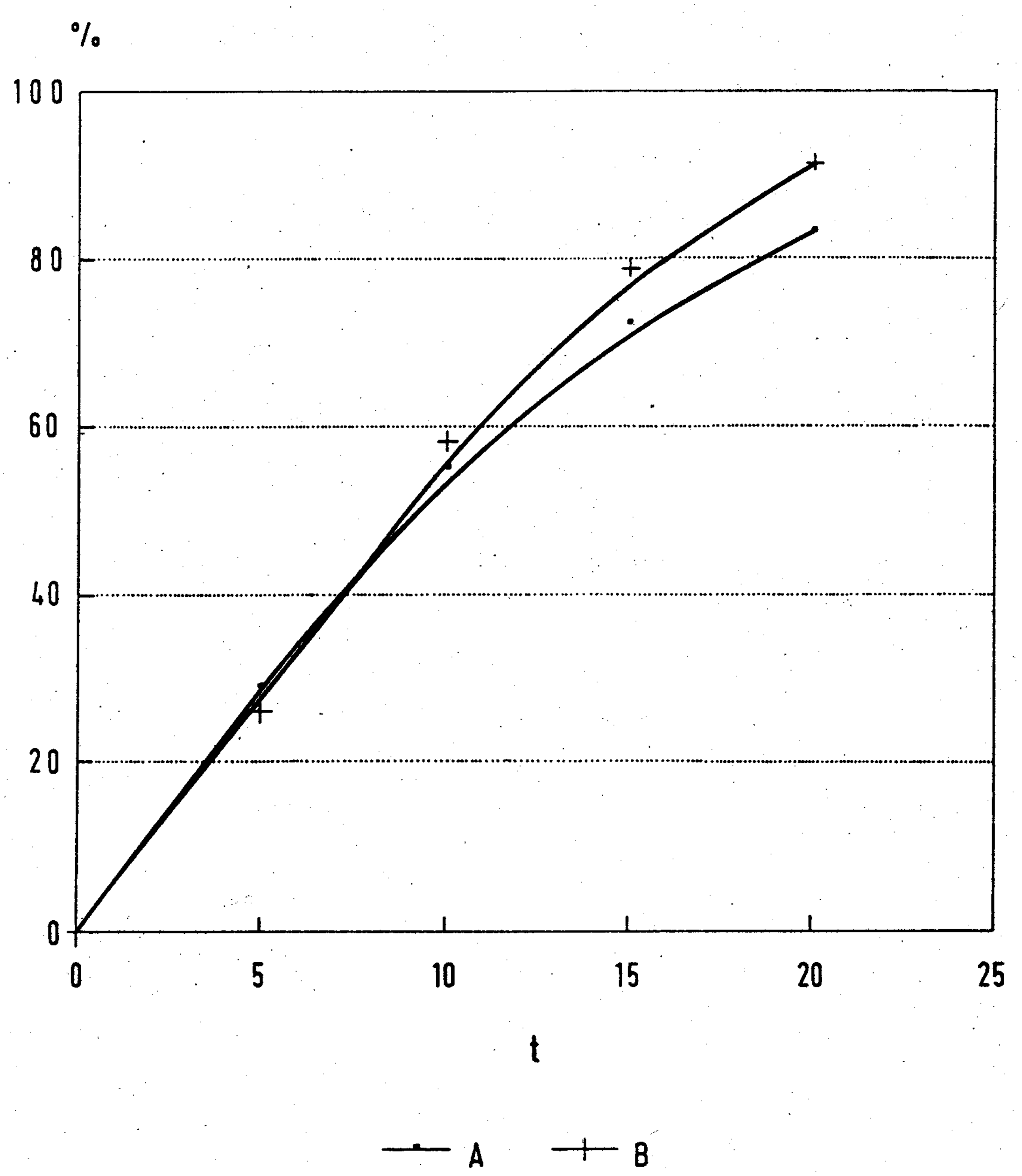


Fig.9

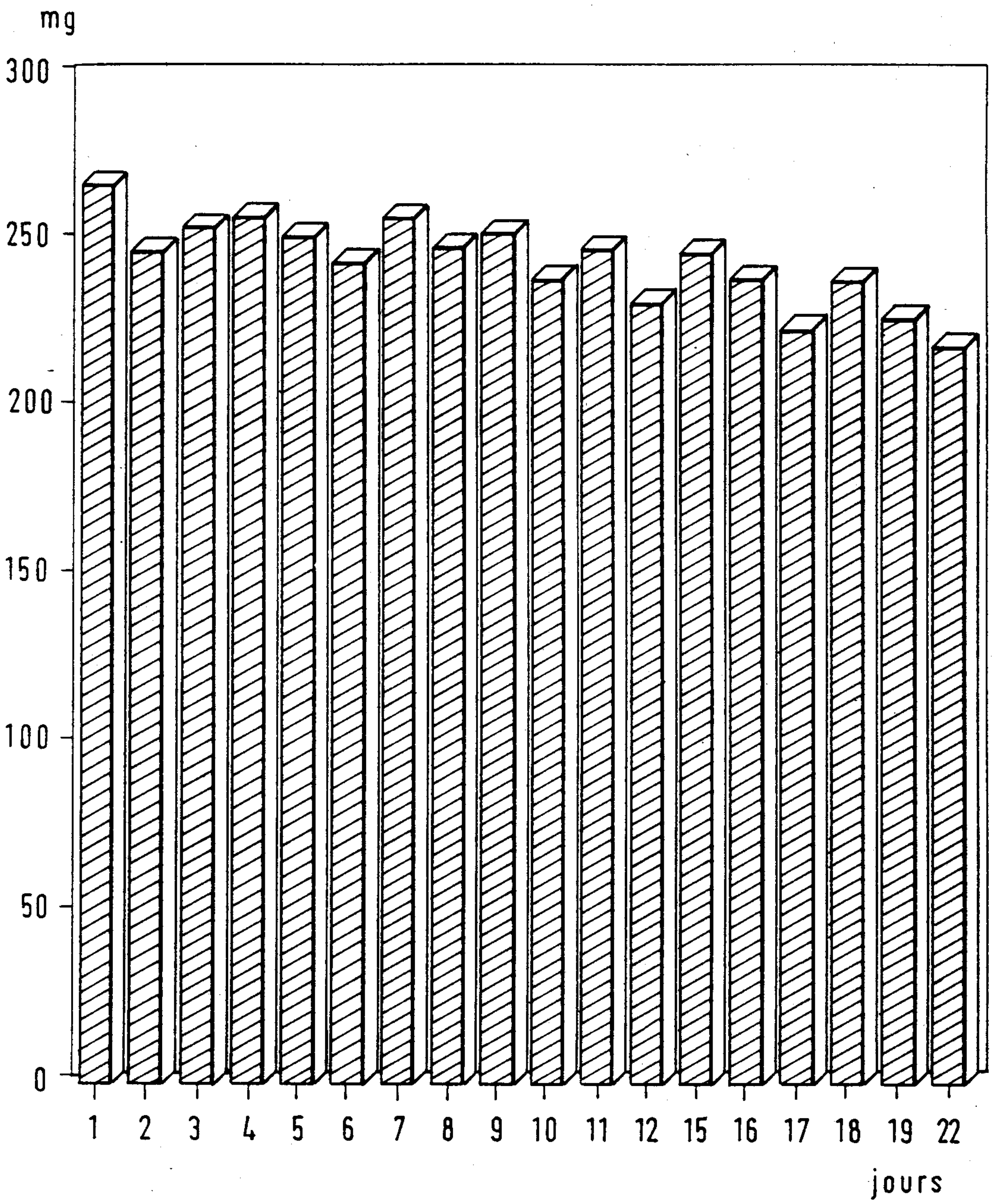
mg
300
250
200
150
100
50
0
1 2 3 4 5 6 7 8 9 10 11 12 15 16 17 18 19 22
jours

Fig. 10